(19) Europäisches Patentamt
European Patent Office
Office européen des brevets

(11) EP 4 635 301 A2

(12) EUROPEAN PATENT APPLICATION

(43) Date of publication:
**22.10.2025 Bulletin 2025/43**

(21) Application number: **25191968.4**

(22) Date of filing: **28.02.2022**

(51) International Patent Classification (IPC):
***A23K 20/20*** *(2016.01)*

(52) Cooperative Patent Classification (CPC):
**C07C 229/76; A23K 10/22; A23K 10/24; A23K 10/30; A23K 20/142; A23K 20/158; A23K 20/174; A23K 20/20; A23K 20/24; A23K 20/26; A23K 20/28; A23K 20/30; A23K 40/10; A23K 40/30; A23K 50/30;** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.04.2021 CN 202110372778**

(62) Document number(s) of the earlier application(s) in accordance with Art. 76 EPC:
**22734769.7 / 4 295 698**

(71) Applicant: **Guangzhou Insighter Biotechnology Co., Ltd**
**Guangzhou, Guangdong 510663 (CN)**

(72) Inventor: **PENG, Xianfeng**
**Guangzhou, 510663 (CN)**

(74) Representative: **Murgitroyd & Company**
**165-169 Scotland Street**
**Glasgow G5 8PL (GB)**

Remarks:
This application was filed on 25-07-2025 as a divisional application to the application mentioned under INID code 62.

# (54) USE OF THE ASPARTIC ACID COPPER COMPLEX IN THE PREPARATION OF AN ANIMAL FEED ADDTIVE

(57) Use of the aspartic acid copper complex in the preparation of an animal feed additive, the aspartic acid copper complex has the structures as shown below:

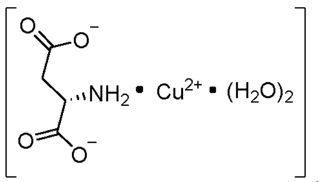

The present aspartic acid copper complex is applied to animal breeding. Calculated in terms of the copper element, a physiological requirement usage amount may promote the growth of livestock and poultry at all stages of the growth cycle, and during high dose use, and the growth performance of the animals is normal under high-dose use, thereby overcoming problems such as excessive use of high-dose inorganic copper in the breeding industry being harmful for animals.

EP 4 635 301 A2

(52) Cooperative Patent Classification (CPC): (Cont.)
**A23K 50/60; A23K 50/75; A23K 50/80**

## Description

### Technical Field

The present application relates to the field of animal feed additives, and specifically to use of the aspartic acid copper complex in the preparation of an animal feed additive.

### Background Art

Copper is one of the essential trace elements for animals. It is a key and active centre of some enzymes, acts as a cofactor of various oxidases, and is a component of coagulation factor V and MT in animals. Copper maintains the normal haematopoietic function, the normal structure of bone, blood vessels and skin, and the health of the central nervous system, protects the normal pigment and structure of the hair, and protects the cells of an organism from the toxicity of superoxide ions. In 1984, K. Kaemmerer reported that copper deficiency could significantly delay the growth of animals, and resupply of copper could quickly restore the growth of animals. The nutritional requirement for copper in animals ranges from 5 mg/kg to 8 mg/kg.

Since studies found in 1945 that the high levels of copper could significantly improve the growth performance of pigs, feeds with 200 mg/kg to 250 mg/kg of high-level copper had been used in the breeding of young piglets to improve their growth performance and shorten the breeding cycle. The availability of inorganic copper is related to its solubility, with higher solubility indicating greater availability. Among the various forms of inorganic copper, copper sulphate is the most preferred due to its comparable effects to organic copper and chelated copper. Therefore, in view of the cost advantage, inorganic copper, especially copper sulphate, has become the main copper source of high-level copper in piglet feeds. However, the high levels of copper have effects similar to antibiotics, significantly promoting growth in piglets bred under conventional conditions, but the high-level copper tends to hinder growth rather than promote it in pigs bred under sterile conditions. The solubility of the inorganic copper source itself or its ability to be rapidly decomposed by gastric acid in the pig's stomach leads to a swift release and absorption of a large amount of free copper ions in the stomach. This exceeds the physiological limit of the animal and leads to toxic reactions. A large amount of copper ions released in gastric acid can be absorbed due to the presence of phytic acid or fibre in the diet, resulting in only a small amount of copper ions reaching the middle and distal parts of the small intestine to perform an antimicrobial effect or be absorbed by the small intestine into an organism to exert their biological effects. Therefore, it is necessary to add a high concentration of copper ions in the feeds to achieve the antimicrobial and growth-promoting effects. Copper level exceeding the physiological limit can lead to numerous side effects, such as organ damage by high-level copper, food safety issues due to excessive residues in organs, interference with the absorption of other nutrients, and environmental pollution caused by unused copper excreted through faeces.

Sources of copper for animal consumption can be broadly divided into two categories: the first category includes inorganic copper, such as copper sulphate, copper chloride, copper oxide, copper acetate, copper carbonate and copper sulphide; the second category includes chelated copper or organic-inorganic copper complex, such as casein copper, milk protein copper, soybean protein copper, methionine copper, copper stearate, and copper lysine hydrochloride. The prior art also shows that as monogastric animals or poultry grow, inorganic copper or organic copper at high levels tends to have reduced effects on the production performance of the animals.

Aspartic acid is not essential for mammals and can be produced from oxaloacetic acid via transamination.

In view of the above, the present application is hereby filed.

### Summary of the Invention

The present application aims to provide use of the aspartic acid copper complex in the preparation of an animal feed additive.

In order to achieve the objectives of the present application, the specific technical solutions are as follows.

Use of the aspartic acid copper complex in the preparation of an animal feed additive, the aspartic acid copper complex has the structures as shown below:

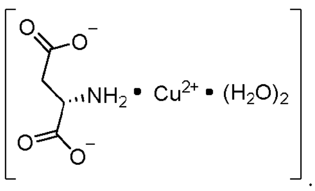

In some technical solutions, the animal is livestock, poultry, aquatic animals or pets at all stages of growth.

In some technical solutions, the animal feed additive is a feed additive suitable for livestock, poultry, aquatic animals, or pets at all stages of growth.

In some technical solutions, the recommended dosage of the aspartic acid copper complex, calculated in terms of copper element, ranges from 5 mg/kg to 300 mg/kg.

In some technical solutions, the aspartic acid copper complex is a copper element supplement and the recommended dosage thereof in an animal feed, calculated in terms of copper element, ranges from 5 mg/kg to 35 mg/kg.

In some technical solutions, the aspartic acid copper complex is a growth promoter for an animal.

In some technical solutions, the recommended dosage of the aspartic acid copper complex in an animal feed, calculated in terms of copper element, ranges from 5 mg/kg to 250 mg/kg, and the animal is a pig, a chicken or a duck at all stages of growth.

Compared with the prior art, the present application has beneficial effects that include:
in the present invention, it is found that the aspartic acid copper complex having a chemical structure of $[(Cu(II))(Asp)(H_2O)_m]\cdot (H_2O)_n$, when applied to animal breeding and calculated in terms of copper element, can promote the growth of livestock and poultry at all stages of the animal's growth even when only physiological necessary quantities are used, and the growth performance of the animals is normal under high-dose use and overcomes problems such as excessive use of high-level inorganic copper in the breeding industry being harmful for animals.

Any embodiment according to any aspect of the present application can be combined with other embodiments, unless they are contradictory to each other. **In** addition, in any embodiment according to any aspect of the present application, any technical feature can be applied to the technical feature in other embodiments, unless they are contradictory to each other.

## Brief Description of the Drawings

FIG. 1 shows the infrared diffraction spectrum of the aspartic acid copper complex having a chemical formula of $[Cu(L\text{-}Asp)(H_2O)_2]$.

FIG. 2 shows the infrared diffraction spectrum of the mixture of the preparation raw materials of the aspartic acid copper complex having a chemical formula of $[Cu(L\text{-}Asp)(H_2O)_2]$.

## Detailed Description of Embodiments

The foregoing content is merely a summary of certain aspects of the present application, but is not limited to these aspects. The content involved above and in other aspects will be described more specifically and completely below.

Certain embodiments of the present application will now be described in detail, examples of which are illustrated by the accompanying structural formulas and chemical formulas. The present application intends to cover all alternative, modified and equivalent technical solutions, which are all included within the scope of the present application as defined by the claims. Additionally, for clarity, some technical features of the present application are described in multiple independent embodiments, respectively, but they may also be provided in combination or in the form of any suitable sub-combination in a single embodiment.

The present invention provides use of the aspartic acid copper complex in the preparation of an animal feed additive.

The term "complex" involved in the present invention refers to a stable substance formed by a copper ion of a certain soluble copper salt, an aspartate ion (chemical structure being $^{-}OOC\text{-}CH_2\text{-}CH(NH_2)\text{-}COO^{-}$, and abbreviated as "Asp" in the present invention) and water molecules in a contact process. The formation of the substance is due to the external and internal conditions that cause them to bind in certain chemical molar equivalent and/or non-chemical equivalent through covalent bonds and/or non-covalent intermolecular forces.

The term "and/or" as used herein comprises any and all combinations of one or more relevant items listed.

Aspartic acid (an English name), also known as asparaginic acid, has a chemical name of aminosuccinic acid and a chemical structure of $HOOC\text{-}CH_2\text{-}CH(NH_2)\text{-}COOH$. Aspartic acid contains asymmetric carbon atoms (denoted as "$\text{-}^{*}CH(NH_2)\text{-}$") and is therefore optically active. Based on the different spatial arrangement positions, aspartic acid can be divided into L-form (

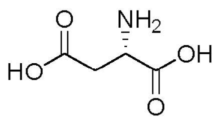
,

formula I) and D-form (

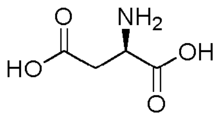

,

formula II), which are enantiomers of each other. D-aspartic acid and L-aspartic acid at equal amounts form an aspartic acid racemate that is not optically active, and the racemate is denoted as DL-form (

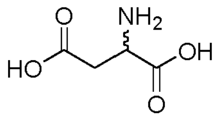

,

formula III). DL-aspartic acid can be an aspartic acid racemate mixture or a racemic aspartic acid. The aspartic acid racemate mixture is a crystalline mixture of equal amounts of D-aspartic acid and L-aspartic acid, and the racemic aspartic acid is a compound formed by D-aspartic acid and L-aspartic acid alternately arranged in the crystal lattices.

The aspartic acid copper complex can be prepared according to the following scheme.

In a preparation scheme, 1 chemical molar equivalent of aspartic acid is added to a 15% (mass in percentage) aqueous solution containing 2 chemical molar equivalents of sodium hydroxide at room temperature. The reaction liquid is stirred to clarification, cooled to room temperature and then slowly added dropwise to a 40% (mass in percentage) aqueous solution containing 1 chemical molar equivalent of copper sulphate pentahydrate. After the dropwise addition, the resulting reaction liquid is further stirred to yield a blue solid. The reaction liquid is filtered, and the filter cake is washed with water and then dried by heating to obtain a solid product.

In one embodiment, the sodium hydroxide can be replaced with an equal molar equivalent of potassium hydroxide.

In one embodiment, the copper sulphate pentahydrate can be replaced with an equal molar equivalent of copper chloride and a hydrate thereof, copper bromide and a hydrate thereof, copper nitrate and a hydrate thereof, etc.

The complex involved in the present invention is an aspartic acid-copper (Cu : Asp = 1 : 1) hydrate. Using structural identification techniques, the inventors have determined that as an integral part of a crystal structure, two water molecules contained in the complex form a stable crystal structure with a copper ion and an Asp.

Further, the reaction liquid is stirred at a low, medium or high stirring rate for 0 to 4 hours.

In some embodiments, the drying by heating is drying under reduced pressure, with the heating temperature of 60°C-110°C and the pressure of the drying oven of 0-0.1 MPa.

In some embodiments, the 1 chemical molar equivalent of aspartic acid is L-aspartic acid.

In some embodiments, the aspartic acid copper complex are used in the preparation of an animal feed additive, wherein the animal feed additive is a livestock feed additive, a poultry feed additive, an aquaculture animal feed additive, or a pet feed additive.

The term "animal" involved in the present invention refers to human or farmed animals that cannot synthesize organic matters from inorganic matters, but can only utilize organic matters as food or feeds to perform vital activities such as ingestion, digestion, absorption, respiration, circulation, excretion, sensation, movement, and reproduction.

Optionally, the farmed animals include poultry, livestock, aquatic animals, and other animals that are raised in captivity and are legally captured including pets. Specifically, the poultry involved in the present invention are edible animals such as chickens, ducks, geese, pigeons, quails or turkeys at all stages of growth; the livestock involved in the present invention are edible animals such as pigs, cattle, goats, rabbits, and horses at all stages of growth; the aquaculture animals involved in the present invention are fish, shrimps, loaches, crabs or eels at all stages of growth; and the pets involved in the present invention include, but are not limited to, cats, dogs, rabbits, etc.

Specifically, the aspartic acid copper complex are used in the preparation of a livestock feed additive, wherein the livestock include but are not limited to, pigs, cattle, goats, horses, rabbits, minks, etc. at all stages of growth.

Specifically, the aspartic acid copper complex are used in the preparation of a poultry feed additive, wherein the poultry include, but are not limited to, chickens, ducks, geese, pigeons, etc. at all stages of growth.

In some embodiments, the animal feed additives prepared from the aspartic acid copper complex are premixed agents, multi premixed agents, aqueous solutions, or granules.

Further, the recommended dosage of the aspartic acid copper complex, calculated in terms of copper element, ranges from 5 mg/kg to 300 mg/kg..

Specifically, the livestock are pigs, cattle, goats, horses, rabbits and minks, preferably pigs, at all stages of growth.

Further, the aspartic acid copper complex is a copper element supplement and the recommended dosage thereof in an animal feed, calculated in terms of copper element, ranges from 5 mg/kg to 35 mg/kg.

Specifically, the poultry are chickens, ducks, geese, pigeons, etc., preferably chickens and ducks, at all stages of growth.

Further, the aspartic acid copper complex is a growth promoter for an animal.

Further, the recommended dosage of the aspartic acid copper complex in an animal feed, calculated in terms of copper element, ranges from 5 mg/kg to 250 mg/kg, and the animal is a pig, a chicken or a duck at all stages of growth.

A skilled person with professional feeding knowledge (referred to as "breeders") know from experience that copper element deficiency in the diet of animals will retard the growth and development of animals, and it is necessary to timely supplement the copper supply to restore the normal growth and development of animals. In light of the animals' need for the copper element, breeders are free to choose different copper sources to feed animals. The copper sources include the aspartic acid copper complex provided by the present invention and the feed composition thereof, feed thereof or feed additive thereof. Breeders provide animals with animal food containing a sufficient amount of the aspartic acid copper complex according to the nutritional requirement for the copper element in animals at all stages of growth.

In some breeding schemes, the animal food includes, but is not limited to, an animal feed, a feed composition, a basal diet, etc.

In some specific breeding examples, the animal is livestock at all stages of growth, preferably pigs at all stages of growth. When provided with animal food comprising the aspartic acid copper complex and containing physiological necessary quantities of copper elements, the experimental pigs show improved food intake, average daily gain and feed conversion compared with the experimental pigs in the copper sulphate breeding example, aspartic acid breeding example or control breeding example without the drug.

In other specific breeding examples, the animal is poultry at all stages of growth, preferably chickens and ducks at all stages of growth. When provided with animal food comprising the aspartic acid copper complex and containing physiological necessary quantities of copper elements, the experimental chickens or ducks show improved feed conversion compared with the experimental chickens or ducks in the copper sulphate breeding example, aspartic acid breeding example or control breeding example, and when the experimental amount of the aspartic acid copper complex reaches 100 to 300 mg/kg, a poisoning phenomenon similar to that observed among the animals in the high-level copper sulphate breeding example is not observed among the experimental chickens.

It can be seen therefrom that in terms of animal growth performance improvement, the aspartic acid copper complex provided by the present invention can not only meet the growth requirement of animals, but also significantly improve the production performance of animals, compared with an inorganic copper source.

The embodiments of the present application will be described in detail with reference to examples below; however, it would be understood by those skilled in the art that the examples below are only used to illustrate the present application, and should not be considered as limiting the scope of the present application. If no specific conditions are indicated in the examples, conventional conditions or the conditions suggested by the manufacturer shall be followed. The reagents or instruments used therein for which manufacturers are not specified are all conventional products that are commercially available.

**Example A** Preparation of aspartic acid copper complex

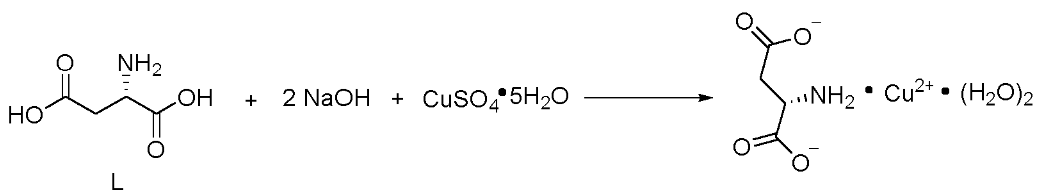


Formula I

Those skilled in the pertinent field will recognize that other methods for preparing the L-aspartic acid copper complex of the present application are considered as falling within the scope of the present application. For example, according to the present application, those unexemplified L-aspartic acid copper complexes can be successfully synthesized by those skilled in the pertinent field by means of modified methods, for example by means of using other reagents or making some conventional modifications to the reaction conditions.

At room temperature, 50 g of L-aspartic acid was added to a reaction flask containing 30.99 g of sodium hydroxide in 200 mL of water. The mixture was stirred to dissolution and clarification, and cooled to room temperature. The prepared

aqueous sodium L-aspartate solution was slowly added dropwise to a reaction flask containing 93.8 g of copper sulphate pentahydrate and 250 mL of water (a clear aqueous solution of copper sulphate pentahydrate). A blue solid was yielded. The aqueous sodium L-aspartate solution was added dropwise over about 1.0 h, and then the reaction liquid was stirred for another 4.0 h and filtered. The filter cake was washed with 100 mL of water and dried at 105°C for 16 h to afford the product (84.6 g, yield: 94.9%) as a blue solid.

Element analysis of product: Cu 26.40%, C 20.89%, H 4.04%, and N 6.07%.

Analysis of infrared diffraction spectrum detection results: FIG. 1 shows the infrared diffraction spectrum detection results of the product. FIG. 2 shows the infrared diffraction spectrum detection results of the mixture obtained by simply mixing the raw materials aspartic acid and copper sulphate pentahydrate, which are required for the preparation of the product in the above-mentioned preparation scheme, according to the feed ratio. It can be seen from the comparison between FIG. 1 and FIG. 2 that there are a significantly reduced number of characteristic absorption peaks in FIG. 1, indicating that the symmetry is higher than that of aspartic acid, and the amino acid characteristic absorption peak at 2083 cm-1 in FIG. 2 is not present in the absorption peaks in FIG. 1; there is a strong and broad absorption peak at 2500 cm-1 to 3400 cm-1 in FIG. 2, whereas there is a relatively narrow absorption peak at 3100 cm-1 to 3400 cm-1 in FIG. 1, indicating that free - OH is not present in the product; and there are coordination characteristic peaks of carboxyl groups at around 1600 cm-1 and 1400 cm-1 in FIG. 1, indicating the coordination between the metal and the carboxyl group.

It is verified by the structural identification technique, element analysis method and infrared diffraction spectrometry that the product obtained using the preparation scheme as shown in this example is an L-aspartic acid copper complex having a structural formula of

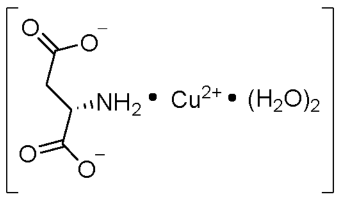

([(Cu(II))(L-Asp)$(H_2O)_2$], compound 1).

Additionally, in some batches, the product from the above-mentioned preparation scheme is dried under reduced pressure at 60°C-140°C. It is verified by the structural identification technique, element analysis method, infrared diffraction spectrometry and thermogravimetric analysis technique that the products obtained further include those having structural formulas of

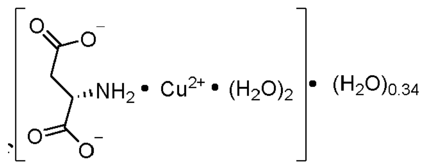

([(Cu(II))(L-Asp)$(H_2O)_2$]· $(H_2O)_{0.34}$, compound 2, blue solid, containing 2.74% non-stoichiometric water) and of

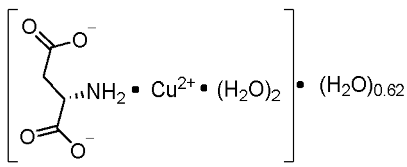

([(Cu(II))(L-Asp)$(H_2O)_2$]· $(H_2O)_{0.62}$, compound 3, blue solid, containing 4.62% non-stoichiometric water). Furthermore, when the product is dried under reduced pressure at 150°C-209°C, it is verified by the structural identification technique, element analysis method, infrared diffraction spectrometry and thermogravimetric analysis technique that the product obtained has a structural formula of

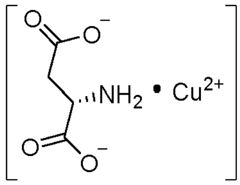

([(Cu(II))(L-Asp)], compound 4, off-white solid).

Moreover, the inventors have found that using an aspartic acid racemate mixture or a racemic aspartic acid in place of L-aspartic acid in the above-mentioned preparation scheme, the following products can be respectively obtained following the preparation processes of compound 1, compound 4 and compound 2:

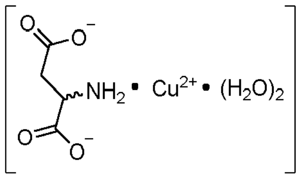

([(Cu(II))(DL-Asp)$(H_2O)_2$], compound 5, blue solid),

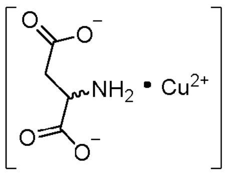

([(Cu(II))(DL-Asp)], compound 6, off-white solid), and

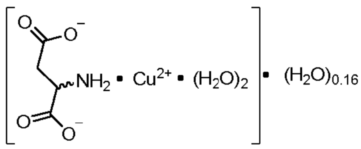

([(Cu(II))(DL-Asp)$(H_2O)_2$]· $(H_2O)_{0.16}$, compound 7, blue solid, containing 1.23% non-stoichiometric water).

Example B Breeding experiment

B-1 Trial for growth-promoting effect of aspartic acid copper complex

(1) Experimental materials

Experimental animals: 900 one-day-old Lingnan yellow-feathered, fast-growing, and large-sized broilers; 150 weaned piglets;

Feeds: basal feed for chickens and basal feed for pigs that contain no antimicrobial drugs, copper sources and growth promoters;

Experimental samples: compound 1, compound 2, compound 3, compound 4, compound 5, compound 6, compound 7,

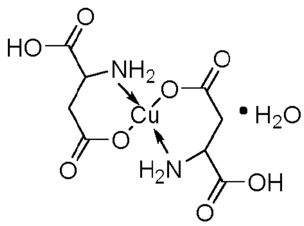

(compound 8, prepared according to the method provided in Chemical World, 2005, 02, 94-96*)*, L-aspartic acid, and copper sulphate pentahydrate.

(2) Experimental method

a. Trial for growth-promoting effect of aspartic acid copper complex in Lingnan yellow-feathered, fast-growing, and large-sized broilers

900 one-day-old Lingnan yellow-feathered, fast-growing, and large-sized broilers were randomly divided into 18 groups (50 broilers/group). In the control group, only a physiological recommendation amount (5 mg/kg) of copper sulphate was added to the feeds; in other groups, different experimental samples as shown in Table 1 were added to the feeds without adding any other copper element supplement. The broilers had free access to food. The weight gain and feed conversion of the experimental chickens (aged from 1 to 21 days) in each experimental group were recorded. Copper sulphate and aspartic acid copper complex were compared in terms of their effects on promoting the growth of broilers.

Table 1 Groups of the trial for growth-promoting effect of aspartic acid copper complex in broilers

| Group | Number of animal | Average initial weight (g) | Growth promoter | Concentration (mg/kg)* |
|---|---|---|---|---|
| Control group | 50 | 50 | Copper sulphate pentahydrate | 5 |
| Copper sulphate pentahydrate-150 | 50 | 50 | Copper sulphate pentahydrate | 150 |
| Copper sulphate pentahydrate-100 | 50 | 50 | Copper sulphate pentahydrate | 100 |
| Copper sulphate pentahydrate-50 | 50 | 50 | Copper sulphate pentahydrate | 50 |
| Compound 1-300 | 50 | 50 | Compound 1 | 300 |
| Compound 1-200 | 50 | 50 | Compound 1 | 200 |
| Compound 1-100 | 50 | 50 | Compound 1 | 100 |
| Compound 1-50 | 50 | 50 | Compound 1 | 50 |
| Compound 1-35 | 50 | 50 | Compound 1 | 35 |
| Compound 1-5 | 50 | 50 | Compound 1 | 5 |
| Compound 2-50 | 50 | 50 | Compound 2 | 50 |
| Compound 3-50 | 50 | 50 | Compound 3 | 50 |
| Compound 4-50 | 50 | 50 | Compound 4 | 50 |
| Compound 5-50 | 50 | 50 | Compound 5 | 50 |
| Compound 6-50 | 50 | 50 | Compound 6 | 50 |
| Compound 7-50 | 50 | 50 | Compound 7 | 50 |
| Compound 8-50 | 50 | 50 | Compound 8 | 50 |
| L-aspartic acid | 50 | 50 | L-aspartic acid | 105 |
| *: different growth promoters were added at amounts calculated in term of copper ion of the compounds, and the amount added in the L-aspartic acid group was consistent with the molar mass of L-aspartic acid contained in the case of the compound 1-50 group. | | | | |

b. Trial for growth-promoting effect of aspartic acid copper complex in pigs

150 weaned piglets were grouped according to Table 2 (10 piglets/group). In the control group, only a physiological recommendation amount (8 mg/kg) of copper sulphate was added to the feeds; in other groups, different experimental samples as shown in Table 2 were added to the feeds without adding any other copper element supplement. The piglets had free access to food. The weight gain and feed conversion of the experimental pigs in each experimental group were recorded within 30 days after weaning. Copper sulphate and aspartic acid copper complex were compared in terms of their effects on promoting the growth of pigs.

Table 2 Groups of the trial for growth-promoting effect of aspartic acid copper complex in pigs

| Group | Number of animal | Average initial weight (kg) | Growth promoter | Concentration (mg/kg)* |
|---|---|---|---|---|
| Control group without growth promoter | 10 | 8.36 | Copper sulphate pentahydrate | 8 |
| Copper sulphate pentahydrate-250 | 10 | 8.31 | Copper sulphate pentahydrate | 250 |
| Compound 1-150 | 10 | 8.42 | Compound 1 | 150 |
| Compound 1-100 | 10 | 8.38 | Compound 1 | 100 |
| Compound 1-50 | 10 | 8.25 | Compound 1 | 50 |
| Compound 1-35 | 10 | 8.31 | Compound 1 | 35 |
| Compound 1-8 | 10 | 8.44 | Compound 1 | 8 |
| Compound 2-50 | 10 | 8.35 | Compound 2 | 50 |
| Compound 3-50 | 10 | 8.32 | Compound 3 | 50 |
| Compound 4-50 | 10 | 8.40 | Compound 4 | 50 |
| Compound 5-50 | 10 | 8.51 | Compound 5 | 50 |
| Compound 6-50 | 10 | 8.37 | Compound 6 | 50 |
| Compound 7-50 | 10 | 8.44 | Compound 7 | 50 |
| Compound 8-50 | 10 | 8.29 | Compound 8 | 50 |
| L-aspartic acid | 10 | 8.43 | L-aspartic acid | 104 |
| *: different growth promoters were added at amounts calculated as the copper ion in the compounds; and the amount added in the L-aspartic acid group was consistent with the molar mass of L-aspartic acid contained in the case of the compound 1-50 group. | | | | |

(3) Experimental results

a. Trial for growth-promoting effect of aspartic acid copper complex in Lingnan yellow-feathered, fast-growing, and large-sized broilers

During the feeding experiment of Lingnan yellow-feathered, fast-growing, and large-sized broilers, in the second week of the experiment, the Lingnan yellow-feathered, fast-growing, and large-sized broilers in copper sulphate groups (150 mg/kg copper ions and 100 mg/kg copper ions, respectively) showed poisoning symptoms such as mussy feathers and dry skin, and by the end of the trial, a small number of chickens died. It indicated that the long-term use of a feed with copper sulphate at an addition amount of 100 mg/kg had toxic side effects on the broilers. In the copper sulphate group using 50 mg/kg copper ions, no clinically visible poisoning symptoms were observed; however, compared with the control group without drugs, during the trial, the group showed a similar relative weight gain rate which was 98.9%, but exhibited a 0.048 higher feed-to-meat ratio, suggesting that the feed conversion was not improved. In the experimental groups of compound 1, compound 2, compound 3, compound 4, compound 5, compound 6 and compound 7, the compounds substantially demonstrated good growth-promoting effects and the experimental chickens exhibited the normal physical characteristics. The relative weight gain rate was increased by 4.9%-19.6%, and the feed conversion was decreased by 2.95%-6.34%. Among them, the experimental group using compound 1 at an addition amount of 50-100 mg/kg exhibited a

dose effect in the experimental results. With regard to the compound 4 group, the relative weight gain rate was increased by 7.2%, and the feed conversion was decreased by 3.23%. With regard to the L-aspartic acid group, the relative weight gain rate was increased by 4.2%, and the feed conversion was decreased by 0.32%. The experimental results suggest that the aspartic acid copper complexes have good safety and growth-promoting effects; however, aspartic acid copper complexes with different structures show different effects on the growth performance of the animals while provided at the same addition amounts (see Table 3 for details).

Table 3 Results of the trial for growth-promoting effect of aspartic acid copper complex in broilers

| Group | Number of animal (broiler/broilers) | Survival rate (%) | Average weight gain (g) | Relative weight gain rate (%) | Average feed consumption (g) | Feed-to-meat ratio |
|---|---|---|---|---|---|---|
| Control group without drug | 50 | 100 | 474 | - | 891 | 1.879 |
| Copper sulphate-150 | 50 | 88 | 427 | 90.1 | 818 | 1.915 |
| Copper sulphate-100 | 50 | 94 | 459 | 96.8 | 881 | 1.920 |
| Copper sulphate-50 | 50 | 100 | 469 | 98.9 | 923 | 1.969 |
| Compound 1-300 | 50 | 100 | 526 | 111.0 | 926 | 1.760 |
| Compound 1-200 | 50 | 100 | 567 | 119.6 | 1004 | 1.771 |
| Compound 1-100 | 50 | 100 | 546 | 115.2 | 970 | 1.777 |
| Compound 1-50 | 50 | 100 | 518 | 109.3 | 923 | 1.782 |
| Compound 1-30 | 50 | 100 | 511 | 107.8 | 919 | 1.798 |
| Compound 1-5 | 50 | 100 | 497 | 104.9 | 916 | 1.843 |
| Compound 2-50 | 50 | 100 | 509 | 107.4 | 908 | 1.783 |
| Compound 3-50 | 50 | 100 | 514 | 108.4 | 919 | 1.788 |
| Compound 4-50 | 50 | 100 | 508 | 107.2 | 924 | 1.819 |
| Compound 5-50 | 50 | 100 | 503 | 106.1 | 917 | 1.824 |
| Compound 6-50 | 50 | 100 | 510 | 107.6 | 912 | 1.789 |
| Compound 7-50 | 50 | 100 | 515 | 108.6 | 921 | 1.789 |
| Compound 8-50 | 50 | 100 | 478 | 100.8 | 895 | 1.872 |
| L-aspartic acid | 50 | 100 | 480 | 101.3 | 899 | 1.873 |

b. Trial for growth-promoting effect of aspartic acid copper complex in pigs

During the feeding trial of pigs, in the high-level copper sulphate pentahydrate group (250 mg/kg), an obvious growth-promoting effect was observed. During the experiment, the average weight gain was increased by 12.66% and the feed conversion was decreased by 7.8% compared with the control group without drugs. Compound 1, compound 2, compound 3, compound 4, compound 5, compound 6, compound 7 and compound 8 were used in this experiment. The first seven compounds all had growth-promoting effects on the experimental pigs. However, compound 1, compound 2, and compound 3, when used at the same doses, achieved about 2-fold improvement in the feed conversion of the experimental pigs compared with compound 4, and compound 1, compound 2, and compound 3 achieved similar feed conversion at lower doses than compound 4. In addition, when the experimental pigs were provided with compound 8 at this dose, their production performance was substantially close to that achieved in the control group. Additionally, compound 1 exhibited a dose effect on improving the production performance of the experimental pigs. When the compound was provided at a dose of 50 mg/kg, its growth-promoting effect and feed conversion rate were close to those achieved in the high-level copper sulphate group (Table 4). The results suggest that 50 mg/kg compound 1 can be used as a substitute for high-level copper sulphate in pig breeding.

Table 4 Results of the trial for growth-promoting effect of aspartic acid copper complex in pigs

| Group | Number of animal (pig/pigs) | Survival rate (%) | Average weight gain (kg) | Relative weight gain rate (%) | Total weight gain (kg) | Total feed consumption (kg) | Feed-to-meat ratio |
|---|---|---|---|---|---|---|---|
| Control group without growth promoter | 10 | 100 | 10.90 | 100 | 109 | 250.28 | 2.296 |
| Copper sulphate pentahydrate-250 | 10 | 100 | 12.28 | 112.66 | 122.8 | 260.09 | 2.118 |
| Compound 1-200 | 10 | 100 | 12.34 | 113.21 | 123.4 | 258.28 | 2.093 |
| Compound 1-100 | 10 | 100 | 12.21 | 112.02 | 122.1 | 257.86 | 2.112 |
| Compound 1-50 | 10 | 100 | 12.12 | 111.19 | 121.2 | 257.08 | 2.121 |
| Compound 1-30 | 10 | 100 | 11.66 | 106.97 | 116.6 | 257.09 | 2.205 |
| Compound 1-8 | 10 | 100 | 11.26 | 103.30 | 112.6 | 255.34 | 2.268 |
| Compound 2-50 | 10 | 100 | 12.30 | 112.8 | 123.0 | 260.21 | 2.116 |
| Compound 3-50 | 10 | 100 | 11.92 | 109.4 | 119.2 | 251.97 | 2.114 |
| Compound 4-50 | 10 | 100 | 11.67 | 107.06 | 116.7 | 257.63 | 2.208 |
| Compound 5-50 | 10 | 100 | 11.83 | 108.5 | 118.3 | 258.35 | 2.184 |
| Compound 6-50 | 10 | 100 | 12.08 | 110.8 | 120.8 | 262.46 | 2.173 |
| Compound 7-50 | 10 | 100 | 12.16 | 111.6 | 121.6 | 267.33 | 2.198 |
| Compound 8-50 | 10 | 100 | 11.06 | 101.5 | 110.6 | 253.37 | 2.291 |
| L-aspartic acid | 10 | 100 | 11.04 | 101.28 | 110.4 | 252.27 | 2.285 |

**B-2** Use of aspartic acid copper complex in aquaculture feeds

(1) Experimental animals and materials

Experimental fishes: The experimental fishes used were black carp (subyearlings), which were provided by the Dafeng fish nursery (Huizhou city, Guangdong province). Healthy and active fingerling black carps (uniform in size) were fed for 4 weeks in a big net cage ($4 \times 2 \times 1.5$ $m^3$) and then the formal breeding experiment was carried out. The experimental systems were small floating net cages ($1.1 \times 1.1 \times 1.1$ $m^3$ in size). Each small net cage was provided with an air-filling head, which allowed for continuous inflation of the cages for 24 h a day. The small net cages and the acclimatization net cage were all placed in a 3500 $m^2$ pond in the experimental field. The pond had a water depth of about 1.5 meters, and the water in the pond was fully aerated underwater. During the trial, 480 black carps (fasted for 1 d) were randomly divided into 10 groups, with 4 replicates for each group and 12 fishes in each replicate. After weighing as a whole, the black carps were randomly placed into 28 net cages and fed with different experimental feeds.

Experimental feeds: The experimental feeds were formulated following the formula in Table 5. According to Table 6, different copper element supplements (calculated in terms of the copper ions) were added to the feeds in different experimental groups, respectively. The feed raw materials were subjected to ultrafine grinding and then granulated into floating expanded feeds (3 mm) using an expansion machine from Jiangsu Muyang Corporation. The demoulding temperature was 130°C. 3% soybean oil was externally sprayed using an oil spraying device. The finished product was sealed and stored in a cool place for later use.

Table 5: Black carp feed formula for trial and chemical ingredients (%wt.)

| Raw material composition | Content (%) | Raw material composition | Content (%) |
|---|---|---|---|
| Fish meal | 9.0 | Soybean oil | 3.0 |
| Casing meal | 3.0 | Phospholipid-rapeseed meal | 9.0 |

(continued)

| Raw material composition | Content (%) | Raw material composition | Content (%) |
|---|---|---|---|
| Soybean meal | 12.0 | Gluten powder | 4.0 |
| Rapeseed meal | 12.0 | Blood cell powder | 2.0 |
| Monosodium glutamate protein | 3.0 | Vc-phosphate | 0.1 |
| Wheat middling | 12.6 | Calcium dihydrogen phosphate | 1.8 |
| Flour | 17.0 | Choline chloride | 0.2 |
| Bentonite | 0.70 | Various vitamins | 0.1 |
| Rice bran | 10.0 | Premixed agent of trace minerals | 0.5 |

(2) Experimental method

Experimental management: During the trial, the carps were manually fed in a restricted dose. The feeding dose was adjusted once a week, and the doses for all groups (based on the initial body weight) were completely consistent. The carps were fed twice a day (at 7:30 and 15:00). The experiment lasted for 8 weeks. During the trial, the water quality was regularly monitored, and throughout the breeding, the water temperature was kept at 26.88 ± 3.08°C, DO > 5.0 mg O $L^{-1}$, pH 7.8, ammonia nitrogen < 0.50 mg N $L^{-1}$, and nitrite-nitrogen < 0.05 mg N $L^{-1}$.

Parametric statistics: During the trial, the carps in each net cage were weighed as a whole one day after discontinuation of feeding, and the average weight gain (g) and feed-to-meat ratio were calculated. The calculation formulas are as follows:

$$\text{Average weight gain (g)} = \text{Average final weight} - \text{Average initial weight};$$

$$\text{Feed-to-meat ratio} = \text{Food intake/ Weight gain of fishes};$$

(3) Experimental results

The results of the trial for growth-promoting effect of the aspartic acid copper complex in fishes can be seen in Table 6. The results show that the aspartic acid copper complex provided by the present invention can significantly improve the daily weight gain and feed-to-meat ratio of the experiment fishes within the range of the physiological requirement amount limits for the animal. Compared with copper sulphate at an equivalent dose, the aspartic acid copper complex provided by the present invention has superior effects in improving the production performance of cultured fishes.

Table 6 Application effects of aspartic acid copper complex in aquaculture feeds

| Group | Number of animal | Additives | Dose (mg/kg) | Average weight gain (g) | Feed-to-meat ratio |
|---|---|---|---|---|---|
| 1 | 48 | Control group | - | 325 | 1.538 |
| 2 | 48 | Copper sulphate | 30 | 343 | 1.458 |
| 3 | 48 | Compound 1 | 30 | 357 | 1.401 |
| 4 | 48 | Compound 2 | 30 | 368 | 1.359 |
| 5 | 48 | Compound 3 | 30 | 361 | 1.385 |
| 6 | 48 | Compound 4 | 30 | 344 | 1.453 |
| 7 | 48 | Compound 5 | 30 | 349 | 1.433 |
| 8 | 48 | Compound 6 | 30 | 353 | 1.416 |
| 9 | 48 | Compound 7 | 30 | 350 | 1.429 |

(continued)

| Group | Number of animal | Additives | Dose (mg/kg) | Average weight gain (g) | Feed-to-meat ratio |
|---|---|---|---|---|---|
| 10 | 48 | Compound 8 | 30 | 338 | 1.479 |
| Notes: the concentration of the copper-containing preparation among the additives was calculated in terms of the copper ions; and the control group was an experimental group without adding any copper-containing preparation. | | | | | |

## Claims

1. Use of the aspartic acid copper complex in the preparation of an animal feed additive, **characterised in that** the aspartic acid copper complex has the structures as shown below:

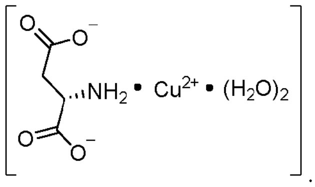

2. The use according to claim 1, **characterised in that** the animal feed additive is a feed additive suitable for livestock, poultry, aquatic animals, or pets at all stages of growth.

3. The use according to claim 1, **characterised in that** the recommended dosage of the aspartic acid copper complex, calculated in terms of copper element, ranges from 5 mg/kg to 300 mg/kg.

4. The use according to claim 3, **characterised in that** the aspartic acid copper complex is a copper element supplement and the recommended dosage thereof in an animal feed, calculated in terms of copper element, ranges from 5 mg/kg to 35 mg/kg.

5. The use according to claim 2, **characterised in that** the aspartic acid copper complex is a growth promoter for an animal.

6. The use according to claim 5, **characterised in that** the recommended dosage of the aspartic acid copper complex in an animal feed, calculated in terms of copper element, ranges from 5 mg/kg to 250 mg/kg, and the animal is a pig, a chicken or a duck at all stages of growth.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Chemical World,*, 2005, vol. 02, 94-96